Europäisches Patentamt

European Patent Office

Office européen des brevets

(19)

(11) Publication number: **0 169 711**
**A2**

(12) **EUROPEAN PATENT APPLICATION**

(21) Application number: 85305135.7

(22) Date of filing: 18.07.85

(51) Int. Cl.⁴: **C 07 C 21/04**
**C 07 C 17/34, B 01 J 23/26**

(30) Priority: 19.07.84 US 632285

(43) Date of publication of application:
29.01.86 Bulletin 86/5

(84) Designated Contracting States:
DE FR GB IT

(71) Applicant: E.I. DU PONT DE NEMOURS AND COMPANY
1007 Market Street
Wilmington Delaware 19898(US)

(72) Inventor: Cohen, Gordon Mark
627 Greythorne Road
Wynnewood Pennsylvania 19096(US)

(72) Inventor: Miller, Kenneth Leron
113 Wood Road
Louisville Kentucky 40222(US)

(74) Representative: Jones, Alan John et al,
CARPMAELS & RANSFORD 43 Bloomsbury Square
London, WC1A 2RA(GB)

(54) Process for the production of 2-chlorobutenes from 2,3-dichlorobutane.

(57) A catalytic process for dehydrochlorination of 2,3-dichlorobutane at about 125-400° in the presence of alumina, alumina gel, or ehromia on alumina produces a mixture of 2-chloro-1-butene and 2-chloro-2-butene, which are intermediates to 2-chloro-1,3-butadiene (or chloroprene), an important monomer for manufacturing certain synthetic elastomers of great commercial interest.

EP 0 169 711 A2

1

## TITLE

### PROCESS FOR THE PRODUCTION OF
### 2-CHLOROBUTENES FROM 2,3-DICHLOROBUTANE

### BACKGROUND OF THE INVENTION

2-Chlorobutenes are known as intermediates in the manufacture of 2-chloro-1,3-butadiene (or chloroprene), which is an important monomer used for making neoprene elastomers. 2-Chlorobutenes can be made, for example, by dehydrochlorinating 2,3-dichlorobutane or 2,2-dichlorobutane with an alkali, such as, for example, sodium hydroxide or calcium hydroxide. They also can be made from 2,2-dichlorobutane by catalytic dehydrochlorination, for example, in the presence of supported alkaline earth metal chlorides, ferric chloride, and copper chlorides, as disclosed in British Patent 1,067,785 to Knapsack AG. Catalytic dehydrochlorination of 2,3-dichlorobutane in the presence of an iron compound and an oxygen-containing gas is described in British Patent 1,266,472 to Denki Kagaku K.K.

For the purpose of the present disclosure, the terms "2-chlorobutene" and "2-chlorobutenes" mean 2-chloro-1-butene, 2-chloro-2-butene, or a mixture of these isomers.

Most of the prior art methods suffer from various disadvantages, which make them impractical to use. Alkali dehydrochlorination presents the obvious shortcoming of requiring a stoichiometric amount of alkali, which is converted to the corresponding chloride and either must be discarded or may be subjected to expensive regeneration, for example, by electrolysis. The earlier catalytic methods either give low yields of the desired product or complex mixtures of several products.

AD-5352

There is, therefore, a need for a more practical catalytic process for the production of 2-chlorobutenes from 2,3-dichlorobutane.

## SUMMARY OF THE INVENTION

According to the present invention, there is provided a process for the catalytic dehydrochlorination of 2,3-dichlorobutane, said process comprising passing gaseous 2,3-dichlorobutane at a temperature of about 125-400°C in contact with a solid catalyst selected from the class consisting of alumina, alumina gel, and chromia on alumina, and recovering from the reaction offgas the products 2-chloro-1-butene, and 2-chloro-2-butene.

## DETAILED DESCRIPTION OF THE INVENTION

The preferred catalyst to be used in the process of this invention is alumina gel, which is partially deactivated. Such partial deactivation occurs when active alumina gel is used in the present process, with the result that the conversion decreases but the yield improves as the initial high activity is partially reduced. Best results were obtained with Alcoa activated alumina gel H-51, 8/14 mesh, deactivated in part by having been used in dehydrohalogenation of 2,3-dichlorobutane.

Other suitable types and grades of alumina include, for example, Woelm activated alumina, N (neutral) and Harshaw Al-1404T.

Activated alumina is a highly porous alumina that adsorbs and holds moisture. Alcoa alumina gel is a high capacity gelatinous alumina, higher than Alcoa activated alumina.

Partial deactivation of alumina gel is believed to involve the removal of certain very active (and nonselective) catalyst sites, thus reducing the overall reaction yields but increasing the selectivity. This can be accomplished either by a physical treatment, such as sintering the catalyst to reduce its surface area, or by a chemical method. Various chemical methods are possible, including a pre-reaction with 2,3-dichlorobutane, treatment with a chemical that destroys some sites such as hydrogen chloride or water, or treatment with a chemical that decomposes with carbonization.

Chromia on alumina may be, for instance, Girdler G-41 from United Catalysts, Inc., having a nominal Cr content of 7.5%.

The starting 2,3-dichlorobutane can be commercial material, or it can be made from 2-butene or from 1,3-butadiene by any convenient method. It can be either the d,l or the meso isomer, or a mixture of both.

The starting material is contacted with the catalyst at any convenient pressure, which may be atmospheric, subatmospheric, or superatmospheric. A convenient temperature range is about 180-330°C.

The reaction products can be conveniently recovered by cooling to about 0°C or less. Since both 2-chlorobutene-2 and 2-chlorobutene-1 can be converted to chloroprene by catalytic oxidation, it is not necessary to separate the 2-chlorobutene mixture into individual isomers.

The process of the present invention does not lead to the formation of significant amounts of 2-chlorobutane, which would be an undesirable side product since it cannot be reused or recycled.

In general, it is more desirable to have a higher selectivity, and thus higher yield of 2-chlorobutenes, even at the cost of lower conversion, than a high conversion and low selectivity.

This invention is now illustrated by the following examples of certain representative embodiments thereof, where all parts, proportions, and percentages are by weight unless otherwise indicated.

EXAMPLE 1 - CONTROL

A quartz tube (33 cm x 2.7 cm I.D.) was packed from the bottom up with: a thin layer of quartz wool, 10 cm length of silica (Fisher, fine granular sand), quartz wool, 5 cm of silica, and quartz wool. At the top of the tube was a head equipped with a thermowell, a gas inlet for introducing nitrogen, and a serum cap-covered inlet for introducing liquid starting material. The tube was heated in a 20 cm electric muffle furnace, the internal reactor temperature being monitored with a thermocouple located in the thermowell in the center of the reagent zone. A syringe pump, a 20 mL syringe, and a long hypodermic needle were used to deliver liquid 2,3-dichlorobutane (2,3-DCBa) to the top layer in the reactor tube. The bottom of the reactor was connected in series to three small tared traps cooled in a dry ice-acetone mixture, and a drying tube.

The reactor was heated to the temperatures shown in Table I. About 10 mL of 2,3-DCBa was added to the syringe, which was then weighed. The reaction was carried out at a 2,3-DCBa flow rate of 0.5 mL/min, with nitrogen diluent at 300 mL/min, until the DCBa was almost all used up. Any traces of product left in the reactor were swept out by means of a 10 minute nitrogen flush at 500 mL/min. The amount of DCBa dispensed was determined by reweighing the syringe, and that of the recovered starting material by reweighing the traps. For analysis by gas chromatography, there was added to each trap chloroform (internal standard) and dichloromethane.

Gas chromatographic (GC) analysis showed that only starting material was recovered. Both GC and weight measurements demonstrated that higher recovery was obtained at higher reactor temperature. Compromising between GC and weight determinations, a 2% value was estimated to be the minimum mechanical loss inherent to the system.

## TABLE I - EXAMPLE 1

| Reactor Temperature | 140°C | 250°C |
|---|---|---|
| 2,3-DCBa dispensed, g | 13.01 | 12.44 |
| 2,3-DCBa recovered, g | | |
| 1st trap | 11.50 | 11.75 |
| 2nd trap | 0.16 | 0.14 |
| 3rd trap | 0.06 | 0.09 |
| Total | 11.72 | 11.98 |
| GC analysis[a] of recovered DCBa, g | | |
| 1st trap | 12.05 | 12.26 |
| 2nd trap | 0.15 | 0.10 |
| Total | 12.20 | 12.36 |
| % recovered | | |
| by wt (3 traps) | 90.1 | 96.3 |
| GC analysis (2 traps) | 93.8 | 99.4 |
| Inherent mechanical loss, % | | |
| by wt | 9.9 | 3.7 |
| GC analysis | 6.2 | 0.6 |

---

(a) Column - 30.5 x 0.32 cm O.D. Carbowax® 20M (Union Carbide) polyethylene glycol and a derivative of terephthalic acid (the proprietary mixture sold under the designation SP-1000 by Supelco, Inc.), 10%, on acid-washed diatomite support

(Johns-Manville Co.). Helium carrier gas at 30 mL/min. Oven temperature program: 60°/8 min, heating rate +32°C/min, plateau at 140°C.

### EXAMPLE 2 - ALCOA ALUMINA GEL AND PARTIAL DEACTIVATION

The reactor described in Example 1 was charged as in Example 1 except that the 10 cm silica layer was replaced by a 10 cm catalyst zone comprising Alcoa activated alumina gel, H-51, 8/14 mesh. The reactor was baked out for 1 hr at 300°C in a 500 mL/min stream of nitrogen. It was then connected in series to two dry ice-cooled traps and a drying tube.

2a. The reactor temperature was lowered to 250°C, and about 10 mL of 2,3-DCBa was introduced at 0.5 mL/min, with an accompanying nitrogen stream at 300 mL/min. In the first 3 min, an exotherm of about 15°C occurred, but the temperature quickly returned to 250°C. The run was followed by a 10 min flush with a 500 mL/min stream of nitrogen. The weight of DCBa dispensed was 10.45 g and the weight of product recovered, 6.17 g. GC analysis gave the product composition shown in Table II. The main products on the column described in Table I were cis- and trans-2-chloro-2-butene and a small amount of 2-chloro-1-butene. Analysis on a second column (30.5 cm x 0.32 cm O.D.), 5% perfluoroalkyl polyether (Du Pont) and 1% $H_3PO_4$ on graphitized carbon (Supelco, Inc.) showed that little or none of the undesired 2-chlorobutane was produced.

Assuming that a 2% mechanical loss of DCBa is inherent to the system, the actual amount of DCBa delivered was calculated to be 10.24 g. Corrected for the mechanical loss, the conversion of DCBa was calculated at 99%, the yield of 2-chloro-2-butenes and 2-chloro-1-butene at 46%.

2b. The reactor was cooled to 200°C and the reaction repeated. No exotherm was observed. The weight of DCBa dispensed was 10.57 g, the weight of Product 10.55 g. GC analysis results are shown in Table II. Corrected for the mechanical loss, conversion was calculated at 18%, yield at 96%.

It can be seen that partial deactivation of the catalyst, which occurs in the initial run, significantly reduces the conversion but also significantly increases the reaction selectivity and thus the yield of 2-chlorobutenes.

### EXAMPLE 3 - ALCOA ALUMINA GEL AND PARTIAL DEACTIVATION

3a. About 10 mL of 2,3-DCBa was dehydrochlorinated in the manner shown in Example 2. The same 10 cm zone of Alcoa alumina gel H-51 was used. The reactor temperature was 200°C and an immediate exotherm occurred when the reaction began. After reaching 216°C in about 6 min, the temperature fell back to 200°C in further 6 min. The run was followed by nitrogen flush. The weight of DCBa dispensed was 11.82 g, the weight of product 9.17 g. GC analysis results are shown in Table II. GC analysis on perfluoroalkyl polyether showed no 2-chlorobutane. Corrected for the 2% mechanical loss, conversion of DCBa was 55%, yield of 2-chloro-2-butenes and 2-chloro-1-butene 61%.

3b. The reaction was repeated at 200°C, and a 3°C exotherm was observed. The weight of DCBa dispensed was 11.47 g, the weight of product 9.47 g. GC analysis results are shown in Table II. The corrected conversion was 20%, the yield 87%.

In this example, neither the conversion nor the yield were outstanding in run 3a. In run 3b, the conversion decreased considerably but the yield increased significantly.

8

### EXAMPLE 4 - WOELM ALUMINA

4a. About 10 mL of 2,3-DCBa was dehydrochlorinated in the manner shown in Example 2. The 10-cm catalyst zone contained Woelm activated alumina N. The reactor temperature was 250°C, and no exotherm occurred. The run was followed by the nitrogen flush. The weight of DCBa dispensed was 11.25 g, the weight of product 5.80 g. GC analysis results are shown in Table II. GC analysis on perfluoroalkyl polyether showed that a very small amount of 2-chlorobutane may have been produced. Corrected for 2% mechanical loss, the conversion of DCBa was 97%, the yield of 2-chloro-2-butenes and 2-chloro-1-butene 47%.

4b. The reaction was repeated at 200°C, and no exotherm was observed. The weight of DCBa dispensed was 11.15 g, the weight of product 7.33 g. GC analysis results are shown in Table II. The conversion was 65%, the yield 36%.

This example shows that activated alumina other than alumina gel is indeed very active in this reaction but not selective (as shown, respectively by the conversions and yields). Pre-reaction, which improved the selectivity of alumina gel in Examples 2 and 3, did not have as significant an effect in this case.

### EXAMPLE 5 - SILICA GEL

This example shows that silica gel gives inferior results.

5a. About 10 ml of 2,3-DCBa was dehydrochlorinated in the manner shown in Example 2. The 10-cm catalyst zone contained silica gel (Fisher, 28/200 mesh). The reactor temperature was 250°C, and no exotherm occurred. The run was followed by the

8

nitrogen flush. The weight of DCBa dispensed was 11.09 g, the weight of product 9.83 g. GC analysis results are shown in Table II. GC analysis on perfluoroalkyl polyether showed little or no 2-chlorobutane. Corrected for 2% mechanical loss, the conversion of DCBa was 15%, the yield of 2-chlorobutenes 35%.

5b. The reaction was repeated at 300°C, and no exotherm was observed. The weight of DCBa dispensed was 11.65 g, the weight of product 8.63 g. GC analysis results are shown in Table II. The corrected conversion was 58%, the yield 30%.

It can be seen that silica gel is a less effective and much less active catalyst than alumina. Run 5b, which produced a higher conversion than run 5a, was carried out at a higher temperature (300°C vs. 250°C).

### EXAMPLE 6 - GIRDLER G-41

About 10 ml of 2,3-DCBa was dehydrochlorinated in the manner shown in Example 2. The 10-cm catalyst zone contained $Cr_2O_3/Al_2O_3$ (Girdler G-41, 7.5% Cr, United Catalysts, Inc.). The reactor temperature was 200°C. A quick exotherm of 50°C occurred, but the temperature fell back in 2-3 min. The run was followed by the nitrogen flush. The weight of DCBa dispensed was 11.80 g, the weight of product 8.09 g. GC analysis results are shown in Table II. GC analysis on perfluoroalkyl polyether showed little or no 2-chlorobutane. Corrected for the 2% mechanical loss, the conversion of DCBa was 54%, the yield of 2-chlorobutene 52%.

The results of Examples 2-6 are summarized in the following Table II.

TABLE II

| Example No. | 2a | 2b | 3a | 3b | 4a | 4b | 5a | 5b | 6 |
|---|---|---|---|---|---|---|---|---|---|
| Catalyst | Alumina Gel | | Alumina Gel | | Woelm Alumina | | Silica Gel | | Chromia/ Alumina |
| Reaction Temperature | 250°C | 200°C | 200°C | 300°C | 250°C | 200°C | 250°C | 300°C | 200°C |
| Recovered, 2,3-DCBa, g | 0.14 | 8.54 | 5.20 | 9.04 | 0.32 | 3.85 | 9.27 | 4.74 | 5.38 |
| 2-chloro-1-butene, g | 0.18 | 0.05 | 0.10 | 0.07 | 0.18 | 0.08 | 0.02 | 0.10 | 0.11 |
| 2-chloro-2-butene, g | 3.10 | 1.20 | 2.66 | 1.31 | 3.41 | 1.76 | 0.38 | 1.32 | 2.18 |
| Conversion, % | 99 | 18 | 55 | 20 | 97 | 65 | 15 | 58 | 54 |
| Yield, % | 46 | 96 | 61 | 87 | 47 | 36 | 35 | 30 | 52 |

## EXAMPLE 7 - GIRDLER G-41

Some $Cr_2O_3/Al_2O_3$ (Girdler G-41, United Catalysts, Inc.) was calcined for 10 min at 700°C. About 375 mL of catalyst was heated to 250-265°C in a reactor tube, and 2,3-DCBa was passed through it at 1.0 g/min. GC analysis showed that DCBa conversion was 89% and 2-chloro-2-butene yield was 96%. The product was identified by comparison of its b.p. (61-2°C) and infrared spectrum with purchased material.

## CLAIMS

1. A process for the continuous catalytic dehydrochlorination of 2,3-dichlorobutane to a mixture of 2-chloro-1-butene and 2-chloro-2-butene, said process comprising contacting 2,3-dichlorobutane at a temperature of about 125-400°C with a solid catalyst selected from the class consisting of alumina gel, alumina, and chromia on alumina, and recovering from the reaction offgas the products 2-chloro-1-butene and 2-chloro-2-butene.

2. A process of Claim 1 wherein the reaction temperature is about 180-330°C.

3. A process of Claim 1 or 2 wherein the catalyst is alumina gel.

4. A process of Claim 3 wherein alumina gel is partly deactivated.

5. A process of Claim 1 or 2 wherein the catalyst is chromia on alumina.

6. A process of Claim 5 wherein the catalyst is calcined prior to the reaction at a temperature of about 700°C.

AD-5352